# EUROPEAN PATENT APPLICATION

(11) **EP 4 524 240 A1**
(43) Date of publication of application: **19.03.2025**
(21) Application number: 23803616.4
(22) Date of filing: 11.05.2023
(51) Int. Cl.: C12N 5/0775, A61L 27/24, A61L 27/36, A61L 27/38, A61L 27/40, A61L 27/50, A61L 27/52, A61L 27/54, C12N 5/10, C12N 15/09

(54) **TENDON/LIGAMENT-LIKE ARTIFICIAL TISSUE PRODUCED USING THREE-DIMENSIONAL MECHANOSIGNALING CELL CULTURE SYSTEM**

(30) Priority: 12.05.2022 JP 2022078641
(71) Applicant: Institute of Science Tokyo, Tokyo 152-8550 (JP)
(72) Inventor: ASAHARA Hiroshi, Tokyo 152-8550 (JP); TSUTSUMI Hiroki, Tokyo 152-8550 (JP); KURIMOTO Ryota, Tokyo 152-8550 (JP)
(74) Representative: Plasseraud IP
(86) International application number: PCT/JP2023/017788
(87) International publication number: WO 2023/219142

(57) **Abstract**

The purpose of the present invention is to provide tendon/ligament-like artificial tissues with sufficient strength. The present invention provides tendon/ligament-like artificial tissues having sufficient strength, by embedding mesodermal stem cells, mesenchymal stem cells, or such in a gel having strength to withstand tension loading, and culturing the cells while applying tension loading to the gel and while adding cells and gel. Human iPS cells, tissue stem cells, or such can be used for mesodermal and mesenchymal stem cells, and such.

## Description

### Technical Field

The present application is an application that benefits from the priority of Japanese Patent Application No. 2022-78641 (filing date: May 12, 2022), which is incorporated herein in its entirety by reference.

The present invention relates to a method for producing a tendon/ligament-like artificial tissue. More specifically, the present invention relates to a tendon/ligament-like artificial tissue produced using a three-dimensional mechanosignal cell culture system. The present invention also relates to a method for producing a tendon/ligament-like artificial tissue using a three-dimensional mechanosignal cell culture system, and a composition used therefor.

### Background Art

Tendons/ligaments are tissues that perform their functions by correctly and strongly connecting muscles and bones, and disorders and diseases of these tendons can significantly impede patients' daily life. In addition, the fact that musculoskeletal disorders are currently a major impediment to healthy life expectancy and account for approximately 20% of caregiving factors among the elderly, comparable to dementia and stroke, makes regeneration and reconstruction of tendons/ligaments an urgent need in the super-aged society of the future.

Furthermore, tendon/ligament injuries are a major factor that threatens the life of athletes. Therefore, regeneration and reconstruction of tendons/ligaments are highly desired in the field of sports medicine.

However, because the tissue-specific master gene has long been unknown, tendon/ligament developmental studies and regenerative medicine have not sufficiently progressed to date.

Analysis of tendon/ligament tissue-specific gene expression patterns identified the transcription factor Mohawk (Mkx), and analysis using Mkx knockout mice revealed that Mkx is essential for tendon/ligament formation (Patent Document 1). The mouse mesoderm stem cell line C3H10T1/2, in which the Mkx gene was transduced by retrovirus, strongly expresses tendon/ligament cell gene markers such as Scleraxis, decorin, and type I collagen compared to the control group (Non-Patent Document 1).

The main component of tendons is an extracellular matrix composed of type I collagen and proteoglycans. However, the formation of structurally meaningful tendon tissue in vivo requires correct orientation of collagen fibers and tendon cells, and simply transplanting Mkx-expressing cells into the body is not sufficient for tissue regeneration in regenerative medicine.

Patent document 2 discloses a method for producing tendon/ligament-like artificial tissues by embedding collagen-secreting cells in a gel having the strength to withstand tension loading and culturing the cells while applying tension loading to the gel.

### Citation List

### Non-Patent Documents

Patent Document 1: JP 2011-205964
Patent Document 2: WO 2019/013279A1

Non-Patent Document 1: Nakamichi R et al. 2016 Nat Commun. 16(7) 12503
Non-Patent Document 2: Kapacee Z et al. 2010 Matrix Biol. 29(8) 668-77
Non-Patent Document 3: Liu H et al. 2015 Stem Cells. 33(2) 443-55
Non-Patent Document 4: Chen X et al. 2012 Sci Rep. 2:977

### Summary of the Invention

### Problems to be Solved by the Invention

One of the objectives of the present invention is to provide a tendon/ligament-like artificial tissue and a method for producing the same.

### Means for Solving the Problems

The present inventors have now developed a new culture method for producing artificial tendons, and have succeeded in producing artificial tendons that are larger and stronger, and have a layered structure similar to biological tendons. Furthermore, it has become possible to produce tissues with sufficient strength not only using cells expressing the Mkx gene but also using mesenchymal stem cells derived from human iPS cells. Tendon injuries, including Achilles tendon injuries, are known to be difficult to recover full motor function because their tissue repair is often delayed. Additionally, shoulder rotator cuff injuries occur in over 60% of people over 70. Surgical repair of tendons and ligaments includes grafting of autologous tissues, and the production and clinical application of artificial tendons has shown promise, but because of the many problems associated with surgical invasion of even healthy areas, there have been no reports of satisfactory results.

In the conventional method of creating artificial tendons, cells and gel are added at the start of culture and the culture is continued. Adding a large amount of cells at once causes cell necrosis and tendons become fragile, which limits the size of the tendons that can be created. Therefore, by introducing tendon cells in stages, artificial tendon tissues are matured in a manner similar to creating annual rings. As a result, a protocol is developed that allows for application to larger sizes and various morphologies, which was previously difficult to achieve. In addition to the conventional introduction of tendon cells, additional gel-embedded tendon cells are added in the middle of culture, and by further continuing the elongation culture, artificial tendons with about twice the width and thickness, respectively, are successfully produced. Tension tests showed that the tension is nearly three times when compared to that by the conventional method.

Furthermore, it was confirmed by scanning electron microscopy that it has a layered structure. Conventional methods have only observed a single-phase structure, whereas the present method has enabled the fabrication of tendon tissue that is more similar to living tissue. In addition, the size and shape of the artificial tendon tissues have been an issue for clinical application to humans, but the method described in this disclosure has shown that it is possible to create artificial tendon tissues of any size and shape. Specifically, when a culture chamber secured with a flat culture space in the center is utilized instead of the long culture chamber used in Non-Patent Document 1, it becomes possible to create an artificial ligament tissue with a 10 mm-wide sheet-like structure, which is about four times wide that by the conventional method. This is expected to have a wide range of applications, including not only tendon grafts for Achilles tendon ruptures, but also patch-type tendon-like tissues for shoulder rotator cuff injuries. In cases where conservative treatment has been the only treatment, the establishment of a minimally invasive treatment method can expand the range of applications and is expected to be applied in many cases.

The present invention is based on such findings and encompasses the embodiments below.
[1] A method for producing a tendon/ligament-like artificial tissue, comprising
   (a) a step of embedding mesodermal stem cells, mesenchymal stem cells, and the like in a gel having strength to withstand tension load, and
   (b) a step of culturing the cells while applying a tension load to the gel, wherein step (b) further comprises a step of culturing by adding cells and the gel.
[2] The method according to [1], which is characterized in that the culture is performed using a culture chamber of an elongated shape in the direction of the stretched longitudinal axis and/or a culture chamber of a planar shape.
[3] The method according to either [1] or [2], wherein the addition of cells and gel is performed multiple times during the culture period.
[4] The method according to any of [1] to [3], which is characterized in that the cells are cultured while increasing the rate of stretching of the tension load.
[5] The method according to any of [1] to [4], which is characterized in that the tension load is at least 1% stretching rate per day.
[6] The method according to any of [1] to [5], wherein the cells are C3H10T1/2 cells, cell lines, normal cells, tissue stem cell-derived cells, ES cell-derived cells, or iPS cell-derived cells.
[7] The method according to any of [1] to [6], wherein the gel is a fibrin gel or a collagen gel.
[8] The method according to any of [1] to [7], wherein the gel comprises a plasmin inhibitor.
[9] The method according to any of [1] to [8], wherein the gel comprises aprotinin.
[10] The method according to any of [1] to [9], further comprising (c) a step of decellularization treatment.
[11] The method according to [10], wherein the decellularization treatment comprises microwave irradiation.
[12] A tendon/ligament-like artificial tissue having a layered structure.
[13] The tendon/ligament-like artificial tissue according to [12], having an annual ring or sheet-like layered structure.
[14] The tendon/ligament-like artificial tissue according to [12] or [13], having an orientation of collagen fibers parallel to the longitudinal direction.
[15] The tendon/ligament-like artificial tissue according to any of [12] to [14], wherein the width in the transverse direction is 2.0 mm or above, 3.0 mm or above, 4.0 mm or above, 5.0 mm or above, 6.0 mm or above, 7.0 mm or above, 8.0 mm or above, 9.0 mm or above, or 10.0 mm or above.
[16] The tendon/ligament-like artificial tissue according to any of [12] to [15], having a tensile strength of at least 0.15 N.
[17] The tendon/ligament-like artificial tissue according to any of [12] to [16], having a tensile strength of at least 0.3 N.
[18] The tendon/ligament-like artificial tissue according to any of [12] to [17], comprising type I collagen.
[19] The tendon/ligament-like artificial tissue according to any of [12] to [18], comprising the cells described in [6].
[20] The tendon/ligament-like artificial tissue produced using the method according to any of [1] to [11] above.
[21] The tendon/ligament-like artificial tissue according to any of [12] to [19] above, which is produced using the method according to any of [1] to [11] above.

### Brief Description of the Drawings

[Figure 1] Figure 1 shows changes in the expression of tendon-related genes, Bgn, Col1a1, Col3a1, and Fmod, as well as tendon/ligament-specific transcription factors, Mkx and Scx, in iPSC-MSCs under stretch-stimulated culture.
[Figure 2A] Figure 2A shows scanning electron microscopy (SEM) images of artificial tendons generated from iPSC-MSCs with (right; Stretch+) and without (left; Stretch-) stretch stimulation. In the case where there is stretch stimulation (stretch+), the orientation of the fibers is more well-defined.
[Figure 2B] Figure 2B shows the distribution of the angle of fiber orientation to the horizontal direction (Angle to Horizontal fiber) for each of the cases with stretch stimulation (Stretch+) and without stretch stimulation (Stretch-). In the case where there is stretch stimulation (Stretch+), the fibers are mostly close to the horizontal direction.
[Figure 3A] Figure 3A shows transmission electron microscope (TEM) images of artificial tendons produced in the conditions with stretch stimulation (right; Stretch+) and without stretch stimulation (left; Stretch-). It can be seen that in the case where there is stretch stimulation (Stretch+), the diameter of the fibers has become larger.
[Figure 3B] Figure 3B shows the distribution of fiber diameters of the artificial tendons produced under the conditions with stretch stimulation (right; Stretch+) and without stretch stimulation (left; Stretch-). It can be seen that the diameter of the fibers in the case with stretch stimulation (Stretch+) (average 15.9 nm) has become larger than that in the case without stretch stimulation (Stretch-) (average 7.9 nm).
[Figure 4] Figure 4 shows a new procedure for the generation of artificial tendons related to the present disclosure. It is shown that after iPSC-MSCs are induced from iPSCs, the gel is added to the iPSC-MSCs and three-dimensional culture is performed. In the novel generation procedure for the present disclosure, cells and gel are added during the course of three-dimensional culture. In the method illustrated in Fig. 3, a 24-hour incubation period (incubation) without tension loading (stretch) is provided at the time of cell and gel addition.
[Figure 5] Figure 5 compares the artificial tendons generated by the new production method of the present disclosure with the one made by the conventional method.
[Figure 6A] Figure 6A shows HE stained images of the axial plane (axial) and sagittal plane (sagittal) of the artificial tendons generated by the new production method.
[Figure 6B] Figure 6B is a SEM image (cross sectional) of an artificial tendon generated by the new production method according to the present disclosure.
[Figure 6C] Figure 6C is a SEM image (superficial layer) of an artificial tendon generated by the new production method according to the present disclosure.
[Figure 7] Figure 7 is a graph showing the results of tension tests of an artificial tendon generated by a conventional three-dimensional mechanosignal cell culture system and a new three-dimensional mechanosignal cell culture system according to the present disclosure.
[Figure 8A] Figure 8A shows the results of the Achilles functional index (AFI) measurement as an evaluation of the recovery of gait function in rats implanted with the artificial tendon generated by the new production method of the present disclosure. Conservation refers to no treatment after Achilles tendon rupture in rats, surgery refers to suture of the Achilles tendon rupture, and transplantation refers to implantation of an artificial tendon at the Achilles tendon rupture site. Normal is the result of normal rats.
[Figure 8B] Figure 8B is a graph showing the AFI results on the day of surgery (d0), one week later (1w), two weeks later (2w), three weeks later (3w), and four weeks later (4w). The AFI value is close to 0 when it is normal, and this means that the value decreases as motor function deteriorates.
[Figure 9] Figure 9 shows changes in tension (N), tension per unit area (MPa), strain rate (%), and Young's modulus (MPa) after transplantation into rats.
[Figure 10] Figure 10 shows three-dimensional culture using a flat culture chamber. The number of cells used was 3.0 x 10⁶.

### Mode for Carrying Out the Invention

As mentioned above, the present inventors have developed a novel culture method for production of artificial tendons, one of the features of which is to mature artificial tendon tissues in a manner similar to creating annual rings by introducing tendon cells in stages, and have succeeded in producing artificial tendons that are larger, stronger, and have a layered structure similar to that of biological tendons. The new artificial tendon tissue of the present disclosure is about twice as wide and thick as conventional artificial tendons, respectively, and tension tests showed that the new artificial tendon tissue is nearly three times wider and thicker when compared with that by the conventional method.

### Tendon/ligament-like artificial tissues

Tendon/ligament-like tissue refers to strong connective tissue mainly composed of type I collagen, which has orientation in vivo. Examples include tendons, ligaments, intervertebral disc fiber rings, and periodontal ligaments.

Tendons are tissues that connect bone to muscle and transmit muscle contraction to bone. Tendon injuries occur due to trauma or aging, but tendons are tissues that lack nutrient blood vessels and are difficult to regenerate, and it is desirable to develop new methods for treating tendon injuries, such as regenerative medicine. The components of tendon are mainly extracellular matrix and tendon cells. The extracellular matrix is produced by tendon cells and contains collagen (collagen type I as the major component, and collagen type III and V as minor collagen, etc.) and proteoglycans (decorin, fibromodulin, biglycan, lumican, etc.).

Ligaments are short bundles of tough connective tissue that connect bone to bone and form joints. Its main component is long collagen fibers, mainly type I collagen. Ligaments also have the function of restricting the range of motion of a joint. Ligaments are similar to tendons and fascia in that they are made of connective tissue, but they are different in the way they connect: ligaments connect one bone to another, tendons connect a muscle to a bone, and fascia connects one muscle to another muscle. All of these are found in the skeletal system of the human body. Ligaments do not usually regenerate spontaneously.

The annulus fibrosus of intervertebral disc is a ligament-like tissue that connects vertebral bodies in the spine. The main components are oriented type I collagen and proteoglycans, similar to those of tendons/ligaments. Since humans walk upright and bipedally, the intervertebral discs are subjected to a steady load of their own weight. Deformation of intervertebral discs caused mainly by aging and excessive pressure is known as intervertebral disc herniation. The only treatment methods available are anti-inflammation with antiinflammatory drugs and surgery to remove the deformed area, and to date, no method has been discovered to regenerate the intervertebral disc.

The periodontal ligament is the connective tissue that connects the tooth root to the alveolar bone. The periodontal ligament has the role of anchoring the tooth to the alveolar bone and relieving the pressure exerted on the tooth during mastication. Its main components are type I collagen and type III collagen, similar to those in tendons/ligaments. It is known that undifferentiated mesenchymal stem cells that have the ability to differentiate into various periodontal tissue cells are engrafted in the periodontal ligament, and regenerative medicine is being considered for the loss of the periodontal ligament due to tooth extraction or periodontal disease.

The tendon/ligament-like artificial tissue of the present invention can be prepared by three-dimensional culture of mesodermal stem cells, mesenchymal stem cells, etc. under tension loading while adding cells and gel. In one embodiment, the tendon/ligament-like artificial tissue according to the present invention has a layered structure. To the best of the inventor's knowledge, such a tendon/ligament-like artificial tissue with a layered structure that more closely resembles living tissue has not been realized before. Thus, in some embodiments, the artificial tissue according to the present disclosure may have a layered structure in the form of annual rings or a layered structure in the form of sheets stacked one above the other. Furthermore, in one embodiment, the tendon/ligament-like artificial tissue according to the present invention has collagen fibers running more densely than in conventional artificial tendons. Furthermore, in one embodiment, the tendon/ligament-like artificial tissue according to the present invention has an orientation of collagen fibers parallel to the longitudinal direction. This orientation of collagen fibers is important for the strength of tendon/ligament-like prosthetic tissues. In addition, the orientation of collagen fibers does not require that all collagen fibers be strictly parallel to the longitudinal direction. Furthermore, in one embodiment, the tendon/ligament-like artificial tissue according to the present invention has a tensile strength of, for example, 0.15N or more, preferably at least 0.3N. The tensile strength can be measured, for example, by a breaking test using a creep meter. Sufficient strength is required for the created tendon/ligament-like artificial tissue to be transplanted into a living body and function. The tendon/ligament-like artificial tissue according to the present invention can be used in a treatment method that comprises a step of transplanting the produced tendon/ligament-like artificial tissue to a human or non-human. The strength can be further increased by bundling a plurality of tendon/ligament-like artificial tissues produced by the method of the present invention. In addition, 3D stretch culture has been performed using mesenchymal stem cells induced with human iPS cells as mesodermal stem cells, mesenchymal stem cells or such, and collagen secretion has been obtained, making it possible to produce artificial tissues.

### Mesodermal stem cells, mesenchymal stem cells and such

In the present culture method, a plurality of cells such as mesodermal stem cells and mesenchymal stem cells can be used. It has been shown that tendon-related gene expression and collagen production are increased by carrying out 3D stretch culture on human iPS cell-derived mesenchymal stem cells, and therefore it is possible to use these cells. The secreted collagen includes at least type I collagen. For example, established strains of mesodermal stem cells (such as mouse C3H10T1/2 cells), mesodermal stem cells derived from a living organism (such as human bone marrow-derived mesodermal stem cells), or mesodermal stem cells derived from pluripotent stem cells such as iPS cells (preferably human iPS cells) can be used as cells. Cells isolated from living tendon and ligament tissues are assumed to be one example of collagen-producing cells. However, it is not limited to these, and any cell known to those skilled in the art can be selected and used as appropriate. The cells can be, for example, C3H10T1/2 cells, cell lines, normal cells, cells derived from tissue stem cells, cells derived from ES cells, or cells derived from iPS cells. As used herein, the term "cell-derived cell" includes the original cell itself; and for example, "tissue stem cell-derived cell" is understood to include the "tissue stem cell" itself.

Furthermore, in one embodiment of the present invention, in the process of manufacturing the tendon/ligament-like artificial tissue, differentiation of cells into mesodermal stem cells is further performed. Induction of differentiation into mesodermal stem cells can be performed using retinoic acid, for example, with reference to the method described in the literature (Takashima Y et al. 2007 Cell. 129(7) 1377-88).

### Cell culture

When producing the tendon/ligament-like artificial tissue according to the present invention, cells can be embedded in a gel and cultured three-dimensionally. The gel can be fibrin gel or collagen gel. The fibrin gel preferably contains protease inhibitors. Examples of protease inhibitors include plasmin inhibitors and elastin inhibitors. As the plasmin inhibitor, for example, aprotinin, α2-antiplasmin, ε-aminocaproic acid, etc. can be used. As the elastin inhibitor, for example, Elastatinal or such can be used. By including a protease inhibitor such as aprotinin in the gel, the strength of the gel can be maintained during cell culture and transplantation into a living body. The concentration of a protease inhibitor such as aprotinin ranges, for example, from 0.1 mg/ml to 10 mg/ml, preferably from 0.3 mg/ml to 3 mg/ml.

When producing the tendon/ligament-like artificial tissue according to the present invention, three-dimensional cell culture can be performed while adding cells and gel as appropriate and applying tension. Therefore, the gel used for cell culture is required to have the strength that can withstand a tension load. A gel having the strength to withstand a tension load can be obtained, for example, by mixing fibrinogen, thrombin, and aprotinin to prepare a fibrin gel containing aprotinin, as described in the Examples of the present application. However, it is not limited to this. In one embodiment, for example, collagen gel is used. In the present specification, a system that cultures cells three-dimensionally (for example, in a hydrogel) while applying a tension load (stretch tension) is referred to as a "three-dimensional mechanosignal cell culture system". Tension loading can be performed using any method known to those skilled in the art, for example, using Sherpa Pro (Menicon Life Sciences) or STB-140 (Strex Co., Ltd.). The tension can be, for example, a stretching rate of 0.2 to 20% per day, preferably a stretching rate of at least 1%. Stretching rate is defined as the rate of increase in chamber width after stretching compared to the pre-stretching state of the three-dimensional culture chamber. More specifically, the calculation is performed using stretching rate (%) = (chamber width after stretching - chamber width before stretching)/(chamber width before stretching × 100). The cell mechanosignal culture time is, for example, at least 8 hours/day, preferably 16 hours/day. The period of cell culture is typically 7 to 30 days. It is preferable to carry out the stretching load while gradually increasing the stretching rate each day. For example, in a 15-day culture period, the stretching rate on Day 0 can be set to 0%, the stretching rate on Day 1 to 1%, the stretching rate on Day 2 to 2%, the stretching rate on Day 3 to 3%, the stretching rate on Day 4 to 4%, and the stretching rate after Day 5 to 5%.

Addition of cells and gel can be made multiple times at any time during the culture period and, for example, additions can be made every other day. Addition of cells and gel may be done at a frequency of, for example, once every 12 hours, every 24 hours, every 48 hours, every 72 hours, or every 96 hours. For example, if additions are made every other day, cells and gel may be added on Day 0, Day 2, Day 4, Day 6, and Day 8 during a 15-day culture period, and the frequency of additions may be adjusted or stopped during the culture period. In addition, culture may be performed using a culture chamber of an elongated shape in the direction of the stretched longitudinal axis and/or a culture chamber of a planar shape, and the chamber selected may be changed according to the shape of the desired artificial tendon. The number of cells added at one time can be, for example, 1.0 × 10⁵ to 1.0 × 10⁷, for example, 1.0 × 10⁶, but it is not limited to these. Further, the amount of gel added at one time can be, for example, 30 to 3000 µl, such as 100 µl, 300 µl, or 500 µl, but it is not limited thereto. The amount of cells and gel added can be changed arbitrarily during the culture period. Moreover, addition of cells and gel can be performed to the entire culture chamber or to a portion thereof. In some embodiments, the tendon/ligament-like artificial tissue according to the present disclosure has a width in the transverse direction of, for example, 2.0 mm or more, 3.0 mm or more, 4.0 mm or more, 5.0 mm or more, 6.0 mm or more, 7.0 mm or more, 8.0 mm or more, 9.0 mm or more, or 10.0 mm or more.

### Decellularization treatment

Tendon/ligament-like artificial tissues created by three-dimensional mechanosignal cell culture can be subjected to a decellularization treatment to eliminate the living cells contained therein. The decellularization treatment can be performed by any method known to those skilled in the art, for example, high hydrostatic pressure treatment, surfactant treatment, microwave irradiation, or such.

### Medium composition

As a medium composition for three-dimensional cell culture, for example, one containing at least fibrinogen and thrombin, and preferably one containing a protease inhibitor can be used. The three-dimensional cell culture medium composition may contain, for example, fibrinogen at 1 mg/ml to 30 mg/ml, aprotinin at 0.1 mg/ml to 10 mg/ml, and/or thrombin at 0.01 mg/ml to 3 mg/ml, and it may preferably contain 5 mg/ml to 10 mg/ml fibrinogen, 0.3 mg/ml to 3 mg/ml aprotinin, and/or 0.1 mg/ml to 1 mg/ml thrombin. The three-dimensional cell culture medium composition used in the present invention may contain additional components to increase the strength of the gel. Furthermore, the three-dimensional cell culture medium composition used in the present invention may further contain general medium components used for cell culture.

The present invention will be specifically described below with reference to Examples, but the present invention is not limited in any way by these.

### Examples

### Example 1: Generation of artificial tendons by differentiation of iPS cells into iPSC-MSCs and culturing under stretch stimulation

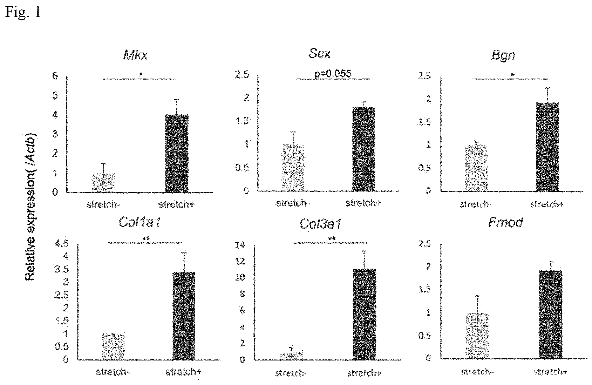

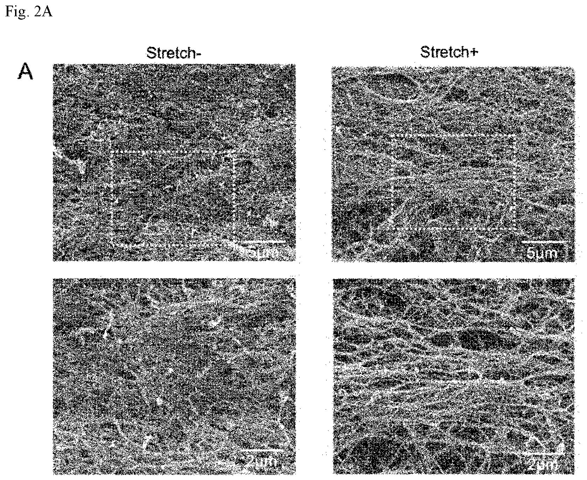

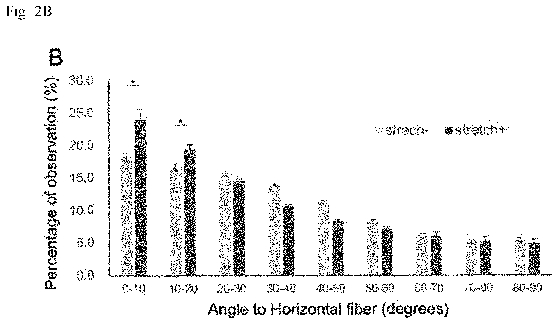

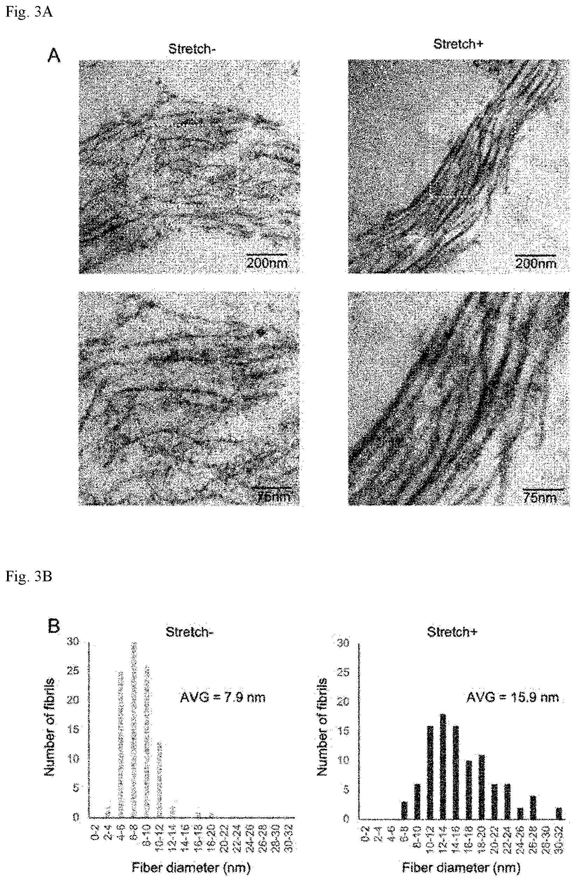

Human iPS cells that exhibit pluripotency and proliferative ability and can express human matrix genes were used as a source to create artificial tendon tissue. First, human iPSCs were induced into neural crest cells (NCCs) with bFGF, SB431542, and CHIR99021 as previously reported (Winston TS., et al, 2019). Thereafter, culture in αMEM was continued to prepare human MSCs (iPSC-MSCs). Based on reports that Mkx expression is induced by mechanical stress and promotes tendon-related gene expression (Kayama et al., 2016; Suzuki et al., 2016), iPSC-MSCs were cultured under 5% stretch stimulation. As a result, the expression of tendon-related genes such as Mkx is increased compared to when no stretch stimulation was applied (Figure 1). Furthermore, in creating an artificial tendon by applying stretching stimulation, it was possible to generate an artificial tendon with collagen fibers that are more parallel and larger in diameter than those without stimulation (Figures 2 and 3). Figure 2 shows the arrangement of fibers along with scanning electron microscopy (SEM) images of an artificial tendon made from iPSC-MSCs, with/without extension stimulation on the left and right, and at 2000x and 5000x magnification on the top and bottom. A 5000x magnified image was taken with SEM at n = 4 and imported into ImageJ, and the direction of the fibers was calculated using the ImageJ plugin, OrientationJ. Figure 3B shows the diameter of the fibers along with a transmission electron microscopy (TEM) image. Images were captured using ImageJ, and the width of collagen was calculated with n = 4. It is thought that the fiber width is wider with stretch stimulation.

### Example 2: Preparation of an artificial tendon derived from iPSC-MSC using a new production method

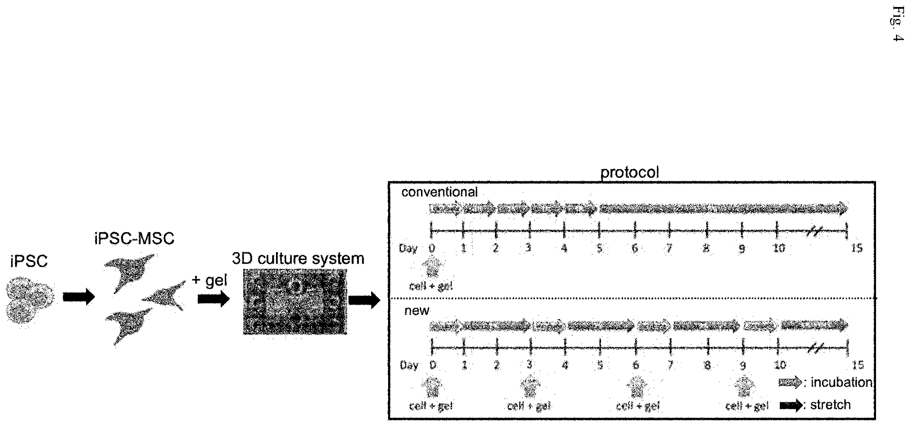

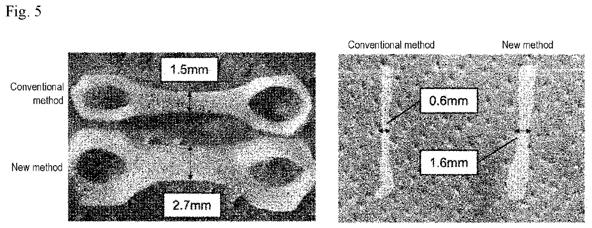

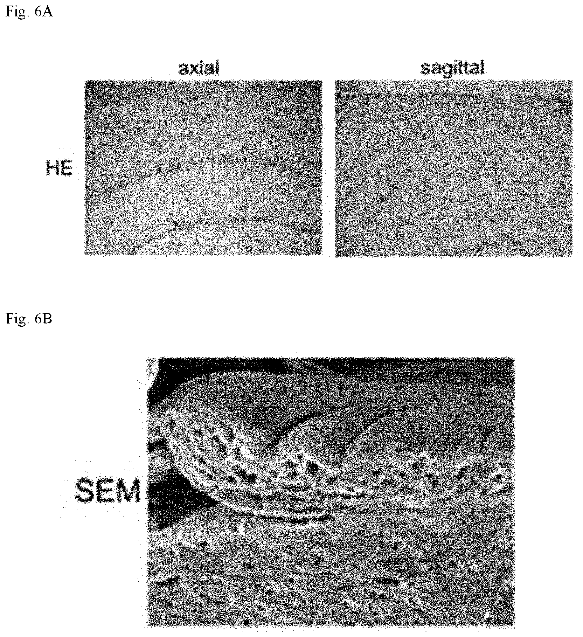

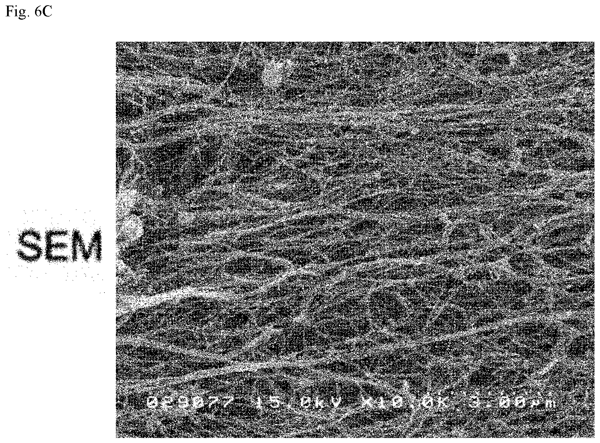

iPSC-MSCs were cultured in 3D stretch culture for 15 days (Figure 4). As shown in Figure 3, in the conventional method, during a 15-day culture period, the stretching rate on Day 0 was set to 0%, the stretching rate on Day 1 was set to 1%, the stretching rate on Day 2 was set to 2%, the stretching rate on Day 3 was set to 3%, the stretching rate on Day 4 was set to 4%, and the stretching rate after Day 5 was set to 5%. On the other hand, in the new production method (new), cells and gel (collagen gel) were added on Day 0, Day 3, Day 6, and Day 9 during the 15-day culture period. The number of cells added at one time was the same as that used at the start of culture (Day 0) in the conventional method, 1.0 × 10⁶, and 300 µl of gel was used. As a result, an artificial tendon-like tissue with uniform thickness was obtained (Figure 5). By adding additional tendon cells embedded in gel during culture and continuing the elongation culture, an artificial tendon was successfully created that is approximately twice as wide and thick as those made using conventional methods. In addition, analysis using HE staining and scanning electron microscopy (SEM) revealed that the artificial tendons generated by the new method are able to increase in size while maintaining the fiber migration seen in the conventional method, and further it was clear that a layered structure was formed (Figure 6).

### Example 3: Decellularization and chemical crosslinking of artificial tendon

In order to eliminate the risk of immune reaction and tumor formation associated with the use of allogeneic human iPS cells, the produced artificial tendon was decellularized. Hyperbaric treatment, an established approach for tissue transplantation (Hashimoto Y., et al, 2010), was used. After decellularization, cells were treated with DNase to suppress nucleic acid-dependent inflammation (Crapo PM., et al, 2011). Chemical cross-linking has been reported to be effective (Makris EA., et al., 2014) for enhancing the mechanical properties of tissues for transplantation (Juncosa-Melvin, N. et al., 2006; Nirmalanandhan VS. et al, 2008). Therefore, to strengthen the mechanical properties of the artificial tendon, chemical crosslinking was performed using N-ethyl-N-(3-(dimethylamino)propyl)carbodiimide/N-hydroxysuccinimide (EDC/NHS).

### Example 4: Tension test of the newly generated artificial tendon

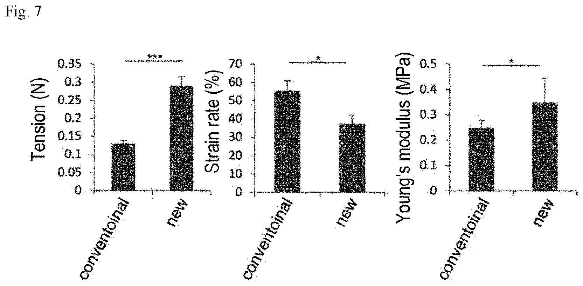

In order to evaluate the mechanical properties of the produced artificial tendon, a tension test was performed (Figure 7). As is clear from the graph in Figure 7, the artificial tendon generated by adding cells and gel and performing 3D stretch culture had a tension nearly three times higher (approximately 0.3N) than that of the artificial tendon produced by performing 3D stretch culture without the addition of cells and gel by the conventional method. Further, improvements in the strain rate (%) and Young's modulus (MPa) were also confirmed. From these results, it is seen that the artificial tendon generated using the new method is wider and has improved physical properties compared to the conventional method.

### Example 5: Transplantation into rats

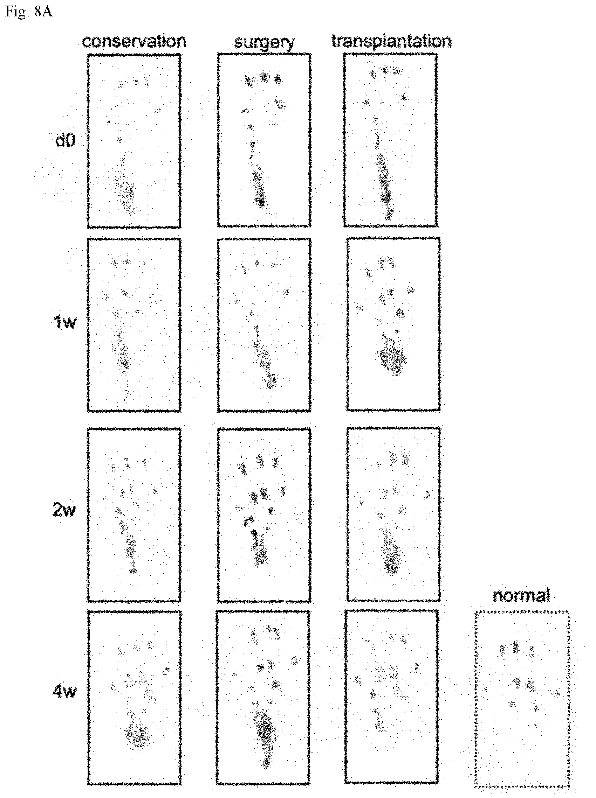

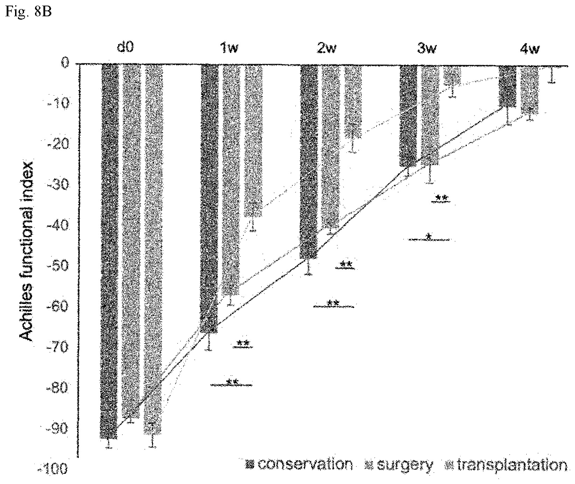

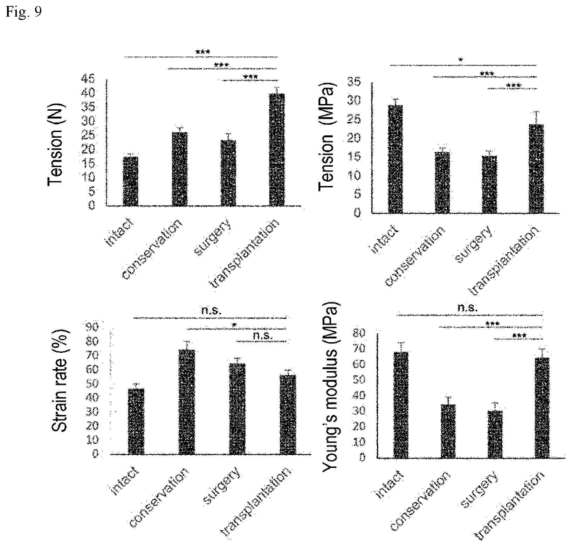

With the creation of a wider artificial tendon, Achilles tendon transplantation into rats was performed. For evaluation of the walking function, the Achilles functional index (AFI) was calculated from the footprints of treated rats. The AFI value is close to 0 when it is normal, and this means that the value decreases as the locomotor function deteriorates. More specifically, the soles of the rat's paws are painted with ink, and the rat is made to walk on a narrow path covered with paper, as shown in the figure on the right. From the rat's pawprint at this time, the length from the first to the fifth toe (paw width (PW)), the length from the second to the fourth toe (intermediate paw width (IT)), and the length from the third fingertip to the wrist joint (footprint length (PL)) were measured on the operated side (E) and the normal side (N), respectively, and the Achilles functional index was calculated using the formula (AFI = 74 x [(NPL- EPL)/EPL] + 161 [(EPW-NPW)/NPW] + 48 x [(EIT-NIT)/NIT] - 5). Lower values reflect a lack of recovery of the Achilles tendon function. Compared to the control conservation therapy group (conservation) and the surgery group (surgery), the transplantation confirmed earlier recovery of the locomotor function (Figure 8). In terms of tension assessment, the results also suggested that artificial tendon transplantation promotes the regeneration of a functional tendon tissue (Fig. 9). Thus, as such, wider and stronger artificial tendons have been successfully generated, and it is thought that the produced artificial tendons promote regeneration of tendon tissue as well as early recovery of the locomotor function in a rat Achilles tendon injury model.

### Example 6: Generation of planar artificial tendon

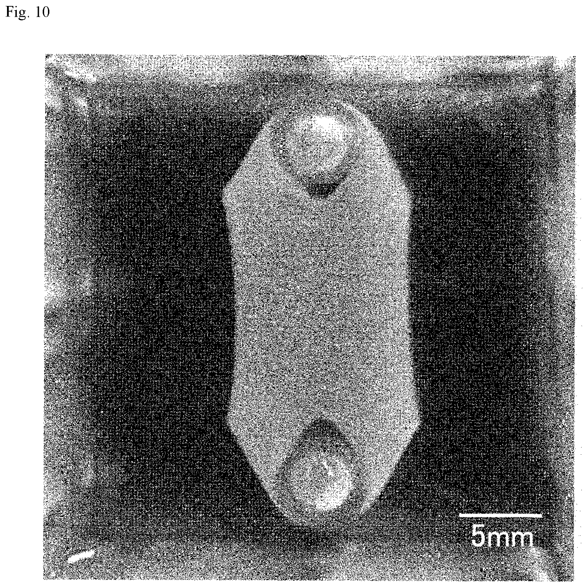

The culture chamber is changed and an iPSC-MSC-derived artificial tendon tissue was produced using the new production method described in the present disclosure. The area of the culture space was increased to ensure a planar culture space. The culture chamber used here allows culture with three times the amount of collagen solution and cells, respectively, compared to the amount of collagen solution and cells cultured in a conventional dumbbell-type chamber. As a result, an artificial tendon tissue with a dense internal structure that is approximately 10 mm wide was successfully generated (Figure 10).

Although preferred embodiments of the invention are shown herein, it is clear to those skilled in the art that such embodiments are provided merely for illustrative purposes, and those skilled in the art may make various variations, changes, and substitutions without departing from the invention. It should be understood that various alternative embodiments of the invention described herein may be used in practicing the invention. In addition, the contents of all publications, including patents and patent application documents referenced herein, should be construed to be incorporated by reference as if they were the same as those specified herein.

### Industrial Applicability

The present inventors succeeded in producing an artificial tissue that has a layered structure and collagen fibers running more densely, which has never been observed in the existing culture systems. By using this new artificial tissue, it is possible to significantly reduce invasiveness compared to autograft, which is the most common existing surgical treatment. Additionally, it can be produced from human iPS cells, thereby avoiding xenogenic immune reactions compared to products derived from animals such as pigs. The present invention enables a regenerative medical approach to tendons/ligaments, and can be extremely useful in treating patients with injured tendons/ligaments.

## Claims

1. A method for producing a tendon/ligament-like artificial tissue, comprising
(a) a step of embedding mesodermal stem cells, mesenchymal stem cells, and the like in a gel having strength to withstand tension load, and
(b) a step of culturing the cells while applying a tension load to the gel, wherein step (b) further comprises a step of culturing by adding cells and the gel.

2. The method according to claim 1, wherein the culture is performed using a culture chamber of an elongated shape in the direction of the stretched longitudinal axis and/or a culture chamber of a planar shape.

3. The method according to either claim 1 or 2, wherein the addition of cells and gel is performed multiple times during the culture period.

4. The method according to any one of claims 1 to 3, wherein the cells are cultured while increasing the rate of stretching of the tension load.

5. The method according to any one of claims 1 to 4, wherein the tension load is at least 1% stretching rate per day.

6. The method according to any one of claims 1 to 5, wherein the cells are C3H10T1/2 cells, cell lines, normal cells, tissue stem cell-derived cells, ES cell-derived cells, or iPS cell-derived cells.

7. The method according to any one of claims 1 to 6, wherein the gel is a fibrin gel or a collagen gel.

8. The method according to any one of claims 1 to 7, wherein the gel comprises a plasmin inhibitor.

9. The method according to any one of claims 1 to 8, wherein the gel comprises aprotinin.

10. The method according to any one of claims 1 to 9, further comprising (c) a step of decellularization treatment.

11. The method according to claim 10, wherein the decellularization treatment comprises microwave irradiation.

12. A tendon/ligament-like artificial tissue having a layered structure.

13. The tendon/ligament-like artificial tissue according to claim 12, having an annual ring or sheet-like layered structure.

14. The tendon/ligament-like artificial tissue according to claim 12 or 13, having an orientation of collagen fibers parallel to the longitudinal direction.

15. The tendon/ligament-like artificial tissue according to any one of claims 12 to 14, wherein the width in the transverse direction is 2.0 mm or above, 3.0 mm or above, 4.0 mm or above, 5.0 mm or above, 6.0 mm or above, 7.0 mm or above, 8.0 mm or above, 9.0 mm or above, or 10.0 mm or above.

16. The tendon/ligament-like artificial tissue according to any one of claims 12 to 15, having a tensile strength of at least 0.15 N.

17. The tendon/ligament-like artificial tissue according to any one of claims 12 to 16, having a tensile strength of at least 0.3 N.

18. The tendon/ligament-like artificial tissue according to any one of claims 12 to 17, comprising type I collagen.

19. The tendon/ligament-like artificial tissue according to any one of claims 12 to 18, comprising the cells according to claim 6.

20. The tendon/ligament-like artificial tissue produced using the method according to any one of claims 1 to 11.

21. The tendon/ligament-like artificial tissue according to any one of claims 12 to 19, wherein the tendon/ligament-like artificial tissue is produced using the method according to any one of claims 1 to 11.
